# FASCICULE DE BREVET EUROPEEN

(11) **EP 3 566 048 B1**
(45) Date de publication et mention de la délivrance du brevet: **24.12.2025**
(21) Numéro de dépôt: 18702316.3
(22) Date de dépôt: 05.01.2018
(51) Int. Cl.: G01N 33/00, G01N 27/417, B64D 37/32

(54) **DISPOSITIF DE MESURE DE LA QUANTITÉ D'OXYGÈNE PRÉSENTE DANS UN GAZ, ET MODULE DE SÉPARATION D'AIR COMPRENANT UN TEL DISPOSITIF DE MESURE**
VORRICHTUNG ZUR MESSUNG DER MENGE DES IN EINEM GAS VORHANDENEN SAUERSTOFFS UND LUFTTRENNUNGSMODUL MIT SOLCH EINER MESSVORRICHTUNG
DEVICE FOR MEASURING THE AMOUNT OF OXYGEN PRESENT IN A GAS, AND AIR-SEPARATION MODULE COMPRISING SUCH A MEASUREMENT DEVICE

(30) Priorité: 09.01.2017 FR 1750185
(43) Date de publication de la demande: 13.11.2019
(73) Titulaire: Safran Aerosystems, 78370 Plaisir (FR)
(72) Inventeur: VANDROUX, Olivier, 38100 Grenoble (FR); GIROUD, Nelly, 42100 Saint-Etienne (FR); PONSINET, Norbert, 78370 Plaisir (FR); GASPAR, Jorge, 93220 Gagny (FR); DELAITRE, Gilles, 95250 Beauchamp (FR); CAZENAVE, Jean-Michel, 38180 Seyssins (FR); BOGGETTO, Philippe, 38360 Sassenage (FR)
(74) Mandataire: Delprat, Olivier
(86) Numéro de dépôt international: PCT/FR2018/050025
(87) Numéro de publication internationale: WO 2018/127667

(56) Documents cités:
- EP-A2- 0 964 246
- DE-A1- 2 608 727
- US-A1- 2015 219 554
- US-B1- 7 385 692
- ANONYMOUS: ""FLAMEPROOF TYPE ZIRCONIA OXYGEN ANALYZER CONVERTER TYPE: ZKME"", April 2011 (2011-04-01), XP093071332, Retrieved from the Internet <URL:https://americas.fujielectric.com/sites/default/files/prod_selector_v/Zirconia%20Oxygen%20Analyzer%20Converter%20%20(Flameproof%20Type)%20Model%20ZKME%20TN1ZMEa-E.pdf> [retrieved on 20230807]
- ANONYMOUS: "FLAMEPROOF TYPE ZIRCONIA OXYGEN ANALYZER CONVERTER TYPE: ZKME", 1 May 2009 (2009-05-01), XP093071293, Retrieved from the Internet <URL:https://www.instrumart.com/assets/Zirconia-ZKME_Manual.pdf> [retrieved on 20230807]
- ANONYMOUS: "THE DIRECT INSERTION TYPE ZIRCONIA OXYGEN ANALYZER DETECTOR TYPE: ZFKE, rev. 1st edition", April 2011 (2011-04-01), XP055398789, Retrieved from the Internet <URL:https://americas.fujielectric.com/files/prod_selector_v/Zirconia Oxygen Analyzer Detector (Flameproof) Model ZFKE TN5ZFKEa-E.pdf?r=false> [retrieved on 20170815]

## Description

### DOMAINE TECHNIQUE

La présente invention se rapporte à un dispositif de mesure de la quantité d'oxygène présente dans un gaz, ainsi qu'à un module de séparation d'air comprenant un tel dispositif de mesure. Par « quantité d'oxygène », on désigne notamment la proportion ou le pourcentage d'oxygène.

L'invention trouve une application avantageuse pour la vérification du pourcentage ou de la pression partielle d'oxygène contenue dans du gaz généré, par exemple, par un système de production de gaz inerte embarqué dans un aéronef, tel qu'un avion.

Une autre application avantageuse se situe dans la mesure du pourcentage d'oxygène présent dans un volume, tel qu'un réservoir de carburant par exemple, pour en vérifier ses propriétés d'inflammabilité.

### ART ANTERIEUR

Dans le domaine de l'aéronautique il est connu des systèmes d'inertage pour réservoirs de carburant d'un aéronef comprenant des modules de séparation d'air comportant des membranes perméables, telles que des membranes en polymère, traversées par un flux d'air. En raison des différentes perméabilités des membranes à l'azote et à l'oxygène, le système divise le flux d'air de telle sorte qu'un flux d'air à forte teneur en azote et un flux d'air à forte teneur en oxygène sont obtenus.

La fraction d'air enrichi en azote est acheminée dans les réservoirs de carburant de l'aéronef de telle sorte que le mélange d'air et de vapeur de kérosène présent dans cet emplacement voit sa concentration en oxygène abaissée pour rendre inerte ledit réservoir.

Alternativement, la fraction de l'air enrichi en oxygène peut être réintroduite dans la cabine des passagers après avoir été traitée avec des moyens appropriés
Dans ces applications, il est important de connaitre précisément la quantité d'oxygène présente dans le gaz rejeté par ledit module de séparation d'air, surtout en ce qui concerne le gaz destiné à rendre inerte un réservoir de carburant.

A cet effet, il est connu d'utiliser un dispositif de mesure mettant en œuvre un moyen de mesure pourvu d'une sonde au zirconium pour effectuer les mesures nécessaires dans ledit gaz pour connaitre la quantité d'oxygène. La sonde au zirconium est notamment alimentée par une tension fixe.

Cependant, ce type de dispositif de mesure est sensible aux conditions environnementales, et la mesure donnée par celui-ci peut dériver de manière incontrôlée. En effet, les mesures effectuées par la sonde au zirconium sont variables en fonction des conditions environnementales d'utilisation de ladite sonde, et notamment en fonction de la température ambiante dans laquelle est maintenu le moyen de mesure.

De plus, la mesure effectuée par la sonde dérive d'une manière aléatoire dans le temps car elle ne prend pas en compte le vieillissement de l'élément sensible à base de zirconium.

Un autre inconvénient réside dans la gestion de la sonde qui ne prend pas non plus en compte les disparités liées à son processus de fabrication.

Comme le décrit le document "THE DIRECT INSERTION TYPE ZIRCONIA OXYGEN ANALYZER DETECTOR TYPE: ZFKE, rev. 1st edition", XP055398789, il existe des sondes en zirconium avec une entrée séparée pour le gaz d'étalonnage et comportant un microcontrôleur capable de piloter des vannes et d'effectuer un auto-étalonnage.

Enfin, dans l'application considérée consistant à analyser un gaz d'inertage, les contrôles de la précision du dispositif de mesure, dans le but de s'assurer que le signal rendu par la sonde n'a pas dérivé, se font d'une manière trop peu fréquente, notamment uniquement lors des opérations de maintenance. De plus l'analyseur décrit possède une fonction permettant de déterminer l'origine des dysfonctionnements éventuels permettant ainsi de gagner du temps lors des opérations de maintenance.

### EXPOSE DE L'INVENTION

L'un des buts de l'invention est donc de remédier à ces inconvénients en proposant un dispositif qui permet de mesurer la quantité d'oxygène présente dans un gaz, d'une manière fiable et précise dans le temps.

Un autre objectif de l'invention est notamment de fournir un tel dispositif de mesure qui ne soit pas sensible aux conditions environnementales, de sorte à limiter la dérive de sa mesure, voire même à la supprimer.

Un autre objectif de l'invention est de fournir un tel dispositif de mesure qui peut être installé en sortie d'un module de séparation d'air d'un système d'inertage de réservoirs de carburant d'un aéronef.

A cet effet, il a été mis au point un dispositif de mesure de la quantité d'oxygène présente dans un gaz à analyser conforme à l'état de la technique en ce qu'il comprend au moins un organe de mesure de la quantité d'oxygène, une première entrée destinée à communiquer avec l'organe de mesure pour l'alimentation en gaz à analyser, et une sortie pour l'échappement dudit gaz analysé.

Conformément à l'invention, le dispositif selon la revendication 1 comprend, entre autres, une deuxième entrée destinée à communiquer avec l'organe de mesure, permettant d'alimenter de façon sélective ledit dispositif avec un gaz étalon présentant une quantité d'oxygène connue, de sorte que la mesure de la quantité d'oxygène présente dans le gaz étalon permette de déterminer une dérive potentielle de la mesure dudit organe de mesure par rapport à la quantité réelle d'oxygène connue présente dans le gaz étalon.

En effet, le gaz étalon permet de vérifier que la mesure donnée par le dispositif de mesure n'est pas altérée et correspond bien à la réalité. Conformément à l'invention, le gaz étalon utilisé est de l'air ambiant dont on sait qu'il est composé, en tout point du globe et jusqu'à une altitude de 15 kilomètres, de 20,9% d'oxygène. De cette manière, par comparaison de la valeur de la quantité d'oxygène réelle présente dans l'air ambiant avec la valeur de la mesure donnée par le dispositif selon l'invention, il est possible d'une part, de vérifier la dérive potentielle de l'organe de mesure et, d'autre part de calibrer ledit organe de mesure pour qu'il indique une mesure exacte.

Le dispositif de mesure selon l'invention comprend un microcontrôleur assujetti à l'organe de mesure pour effectuer un recalibrage automatique de l'organe de mesure en fonction de la dérive déterminée. Cette opération peut se faire en temps réel et à tout moment, d'une manière simple et rapide. Ainsi, il n'est pas nécessaire d'attendre les opérations de maintenance pour contrôler la dérive de l'organe de mesure et de le re-calibrer le cas échéant. Le dispositif de mesure selon l'invention possède donc une précision de mesure optimale sur toute la gamme d'utilisation.

Selon l'invention, le dispositif de mesure selon l'invention comprend un module de sélection piloté par le microcontrôleur et apte à mettre en communication, au choix, la première ou la deuxième entrée avec l'organe de mesure pour analyser soit le gaz provenant de la première entrée, soit le gaz étalon provenant de la deuxième entrée.

Selon une forme de réalisation spécifique, le module de sélection, de préférence pneumatique, comprend une électrovanne comprenant deux entrées, respectivement reliées à la première et à la deuxième entrée du dispositif de mesure, et une sortie en communication avec l'organe de mesure.

Conformément à l'invention, l'organe de mesure de la quantité d'oxygène comprend une sonde au zirconium apte à mesurer la pression partielle d'oxygène présente dans un gaz, de laquelle peut être déduite la quantité d'oxygène présente dans le gaz.

Le dispositif comprend un capteur de température agencé au niveau de la sonde au zirconium et assujetti au microcontrôleur, de sorte que le microcontrôleur est apte à faire varier la tension d'alimentation de la sonde au zirconium en fonction de la température mesurée pour conserver le cœur de la sonde à température constante.

D'une manière avantageuse, le dispositif selon l'invention comprend un capteur de pression agencé au niveau de la sonde au zirconium pour mesurer la pression au point de mesure. Ledit capteur de pression est assujetti au microcontrôleur pour permettre audit microcontrôleur de calculer le pourcentage d'oxygène présent dans le gaz à partir de la pression partielle d'oxygène.

L'invention vise également à fournir un module de séparation d'air pour générateur de gaz d'inertage dans un système d'inertage d'au moins un réservoir de carburant d'un aéronef. Le module présente intérieurement au moins une membrane perméable, et comprend une entrée d'air comprimé destiné à traverser la membrane, une sortie d'air enrichi en oxygène, et une sortie d'air appauvri en oxygène dit gaz d'inertage. Selon l'invention, le module de séparation d'air comprend un dispositif de mesure conforme aux caractéristiques précitées, dont la première entrée est reliée à la sortie d'air appauvri en oxygène du module de séparation d'air, et dont la deuxième entrée est reliée ou destinée à être reliée à une source de gaz étalon.

### DESCRIPTION SOMMAIRE DES FIGURES

D'autres avantages et caractéristiques ressortiront mieux de la description qui va suivre, donnée à titre d'exemple non limitatif, du dispositif de mesure selon l'invention, à partir des dessins annexés dans lesquels :
- la figure 1 est une représentation schématique illustrant en détail le schéma de fonctionnement du dispositif de mesure selon l'invention ;
- la figure 2 est une représentation schématique illustrant la liaison entre le module de sélection pneumatique et l'organe de mesure du dispositif de mesure selon l'invention, pour la mesure de la quantité d'oxygène présente dans un gaz provenant par exemple d'un module de séparation d'air ;
- la figure 3 est une représentation schématique similaire à celle de la figure 2, pour la mesure de la quantité d'oxygène présente dans un gaz étalon, tel que de l'air ambiant par exemple ;
- la figure 4 est une représentation schématique d'un module de séparation d'air selon l'invention, comprenant un dispositif de mesure de la quantité d'oxygène.

### DESCRIPTION DETAILLEE DE L'INVENTION

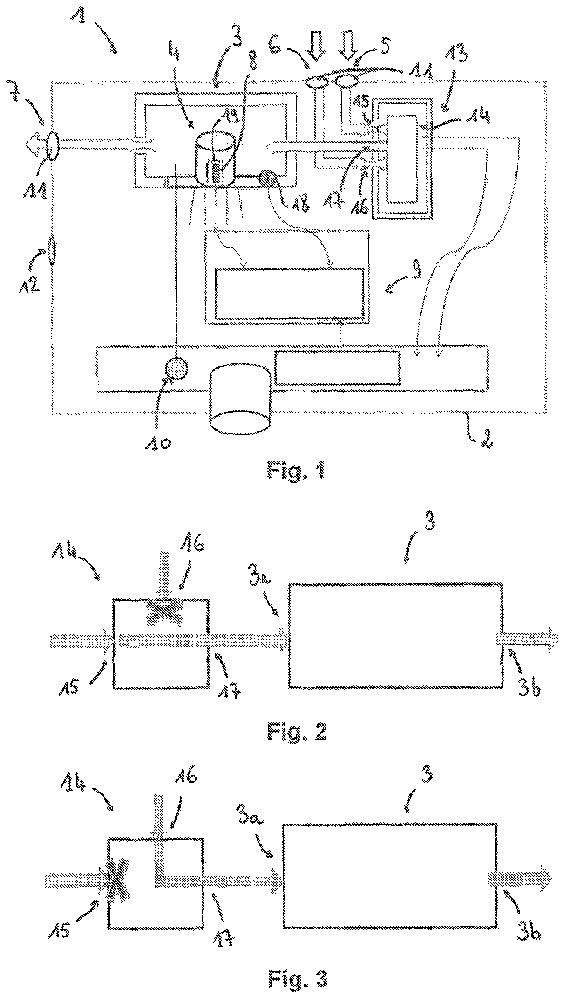

En référence à la figure 1, l'invention concerne un dispositif de mesure (1) de la quantité d'oxygène présente dans un gaz, et trouve une application avantageuse pour la mesure de la quantité d'oxygène présente dans un gaz rejeté par un module de séparation d'air d'un système d'inertage d'un aéronef.

Le dispositif de mesure (1) selon l'invention comprend, par exemple, un boîtier (2) renfermant au moins un organe de mesure (3) de la quantité d'oxygène comprenant par exemple une sonde au zirconium (4). Le boîtier (2) comprend une première entrée (5) destinée à communiquer avec ladite sonde (4) pour l'alimentation en gaz à analyser, une deuxième entrée (6) destinée à communiquer de façon sélective avec la sonde (4) pour l'alimentation avec un gaz étalon, tel que de l'air ambiant, présentant une quantité d'oxygène connue, et une sortie (7) pour l'échappement dudit gaz analysé.

L'organe de mesure (3) comprend à cet effet, une entrée (3a) destinée à être alimenté en gaz et une sortie d'échappement (3b), reliée à la sortie (7) du boîtier (2). D'une manière connue, la sonde (4) comprend une cellule de détection (8) réalisée à partir de zirconium stabilisé et met en œuvre des électrodes pour la mesure de la pression partielle d'oxygène présente dans le gaz. La sonde au zirconium (4) est bien connue de l'état de la technique, par exemple du type KGZ10, et est alimentée par un microcontrôleur (9) avec une tension continue de l'ordre de 4,5 V. Pour déduire le pourcentage d'oxygène présent dans le gaz, le dispositif (1) selon l'invention comprend un capteur de pression (10) agencé au niveau de la sonde au zirconium (4) pour mesurer la pression au point de mesure, et assujetti au microcontrôleur (9) pour permettre le calcul du pourcentage d'oxygène à partir de la pression partielle d'oxygène.

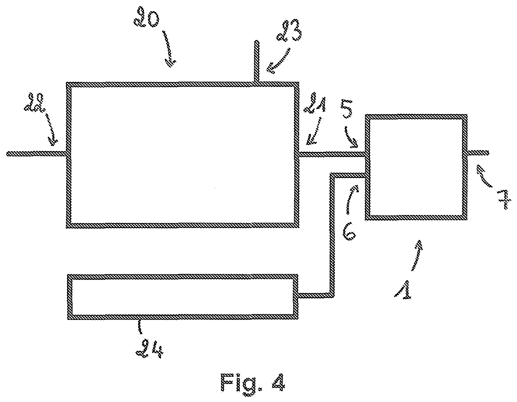

En pratique, en référence à la figure 4, et dans l'application avantageuse considérée, le dispositif de mesure (1) est connecté, par l'intermédiaire de la première entrée (5), à une sortie (21) de gaz d'inertage d'un module de séparation d'air (20) destiné, par exemple, à alimenter en gaz appauvri en oxygène un réservoir de carburant pour le rendre inerte. Plus précisément, le module de séparation d'air (20) présente intérieurement au moins une membrane perméable, et comprend une entrée (22) d'air comprimé destiné à traverser la membrane, une sortie (23) d'air enrichi en oxygène, et la sortie (21) d'air appauvri en oxygène dit gaz d'inertage. Le flux de gaz appauvri en oxygène traverse le dispositif (1) en passant par l'entrée (5), entre en communication avec la sonde au zirconium (4) pour la mesure en tant que telle et par l'intermédiaire de l'entrée (3a) de l'organe de mesure (3), et est ensuite évacué par la sortie de l'organe de mesure (3b) et par la sortie (7) du dispositif (1). La mesure de la quantité d'oxygène présente dans ce gaz est analysée en continu et en temps réel. Dans cette application, les entrées (5, 6) et la sortie (7) du dispositif de mesure (1) comprennent des filtres et des éléments pour stopper la progression des flammes (11). Le dispositif (1) comprend également un orifice de drainage (12) permettant de maintenir une pression constante à l'intérieur du dispositif de mesure (1) et ne pas fausser la mesure.

En pratique, lorsqu'il convient de vérifier la dérive de la mesure donnée par le dispositif de mesure (1), et notamment pour basculer sur la deuxième entrée (6) reliée à une source de gaz étalon (24) tel que de l'air ambiant, le dispositif (1) comprend un module de sélection de préférence pneumatique (13) comprenant une électrovanne (14) comprenant deux entrées (15, 16), respectivement reliées à la première (5) et à la deuxième entrée (6) du dispositif (1), et une sortie (17) en communication avec la sonde au zirconium (4).

En référence aux figures 2 et 3, l'électrovanne (14) est pilotée par le microcontrôleur (9) pour obturer la première entrée (15) ou la deuxième entrée (16) de l'électrovanne (14), et ouvrir l'autre pour mettre en communication la deuxième entrée (6) ou la première entrée (5) du dispositif (1) avec la sonde au zirconium (4).

Le microcontrôleur (9) est donc apte à mettre en communication la deuxième entrée (6) du dispositif de mesure (1) avec la sonde (4) pour effectuer des mesures ponctuelles de la quantité d'oxygène présente dans le gaz étalon pour permette de déterminer une dérive potentielle de la sonde (4) par rapport à la quantité réelle d'oxygène connue présente dans le gaz étalon. En effet, par comparaison de la mesure obtenue avec la quantité réelle connue, à savoir pour l'air ambiant 20,9% d'oxygène en tout point du globe et jusqu'à 15 km d'altitude, le microcontrôleur (9) détermine une dérive potentielle de la mesure de la sonde au zirconium (4).

Le microcontrôleur (9) est assujetti à la sonde au zirconium (4) pour permettre d'effectuer un recalibrage automatique de ladite sonde (4) en fonction de la dérive déterminée. Le microcontrôleur (9) applique, par exemple, une pondération à la valeur mesurée par la sonde au zirconium (4) pour réajuster ladite mesure à la valeur réelle connue. La sonde (4) est donc re-calibrée de manière automatique, en temps réel et sans nécessiter une opération de maintenance.

Pour optimiser la précision de la mesure, le dispositif (1) comprend un capteur de température (18) agencé au niveau de la sonde au zirconium (4) et assujetti au microcontrôleur (9), de sorte que le microcontrôleur (9) est apte à faire varier la tension d'alimentation de la sonde au zirconium (4) en fonction de la température mesurée, notamment pour conserver le cœur de la sonde à température constante. A cet effet, une résistance (19) permet de réchauffer la sonde le cas échéant.

Ainsi, le dispositif de mesure (1) selon l'invention permet, d'une part, de faire varier la tension d'alimentation en fonction de la température ambiante et des caractéristiques individuelles de la sonde (4). Cette gestion a pour effet de conserver le cœur de la sonde à température constante afin d'améliorer la précision de la mesure et de ne pas être sensible aux conditions environnementales et, d'autre part, de re-calibrer la mesure si nécessaire en cours de fonctionnement.

La mesure fournit une pression partielle d'oxygène, qui est utilisée comme telle pour des applications OBOGS, selon l'acronyme anglo-saxon *« On Board Oxygen Generating »,* qui concernent notamment la mise en œuvre de systèmes autonomes de génération d'oxygène respiré, à partir d'un prélèvement d'air moteur, mais qui est transformée en pourcentage d'oxygène par l'intermédiaire du capteur de pression absolu (10) placé à proximité de la sonde (4), pour des applications OBIGGS, selon l'acronyme anglo-saxon *« On Board Inert Gas Generation Systems »,* qui concernent la mise en œuvre de systèmes de génération de gaz inerte pour l'inertage de réservoirs de carburant par exemple.

Toutes les logiques de commandes et de contrôles sont réalisées à partir du microcontrôleur (9) intégré dans le dispositif (1) selon l'invention. Ce microcontrôleur (9) est aussi utilisé pour générer des signaux d'alarme ou de bon fonctionnement des différents composants du dispositif de mesure (1).

Le microcontrôleur (9) est associé à un logiciel qui embarque des courbes de corrections caractérisant au moins une dizaine de sondes au zirconium (4) par exemple, testées dans des conditions environnementales variables pour connaitre la réponse moyenne desdites sondes en fonction de cycles différents de températures ambiantes, de pressions ambiantes, de pressions d'alimentation, et de teneurs en oxygène. Ces courbes sont intégrées dans le dispositif (1) et permettent de corriger la dérive du dispositif (1) le cas échéant.

Le logiciel intègre également des données relatives à des essais de vieillissement de la sonde pour déterminer sa dérive naturelle et permettre d'intégrer une fonction d'autocontrôle et d'auto-calibration. Le logiciel comprend notamment des lois de calibration et d'adaptation du pilotage de la sonde.

Le microcontrôleur (9) permet également de piloter l'électrovanne (14) pour récupérer par exemple, à intervalles réguliers, des mesures de la quantité d'oxygène présente dans le gaz étalon et réalise, à partir desdites mesures, un graphe de la dérive de la sonde au zirconium (4) en fonction du temps.

Ainsi, il ressort de ce qui précède que le dispositif de mesure (1) selon l'invention permet de mesurer la quantité d'oxygène présente dans un gaz, d'une manière fiable et précise, en n'étant pas sensible aux conditions environnementales, de sorte à limiter la dérive de sa mesure, voire même à la supprimer, et permettant un recalibrage automatique et en temps réel de la mesure, limitant de fait les opérations de maintenance.

## Revendications

1. Dispositif (1) de mesure de la quantité d'oxygène présente dans un gaz à analyser, ledit dispositif (1) comprenant un boîtier (2) renfermant au moins un organe de mesure (3) de la quantité d'oxygène comprenant une sonde au zirconium (4) apte à mesurer la pression partielle d'oxygène présente dans un gaz, le boîtier (2) comprenant une première entrée (5) destinée à communiquer avec ladite sonde (4) de l'organe de mesure (3) pour l'alimentation en gaz à analyser, une sortie (7) pour l'échappement dudit gaz analysé, une deuxième entrée (6) destinée à communiquer avec la sonde (4) de l'organe de mesure (3), permettant d'alimenter de façon sélective ledit dispositif (1) avec de l'air ambiant présentant une quantité d'oxygène connue, de sorte que la mesure de la quantité d'oxygène présente dans l'air ambiant permet de déterminer une dérive potentielle de la mesure dudit organe de mesure (3) par rapport à la quantité réelle d'oxygène connue présente dans l'air ambiant, l'organe de mesure (3), **caractérisé en ce que** le dispositif (1) comprend un microcontrôleur (9) assujetti à l'organe de mesure (3) pour effectuer un recalibrage automatique de l'organe de mesure (3) en fonction de la dérive déterminée, le dispositif (1) comprenant un capteur de température (18) agencé au niveau de la sonde au zirconium (4) et assujetti au microcontrôleur (9), de sorte que le microcontrôleur (9) est apte à faire varier la tension d'alimentation de la sonde au zirconium (4) en fonction de la température mesurée, le dispositif comprend en outre un module de sélection (13) piloté par le microcontrôleur (9) et apte à mettre en communication, au choix, la première entrée (5) ou la deuxième entrée (6) avec l'organe de mesure (3) pour analyser soit le gaz provenant de la première entrée (5), soit l'air ambiant provenant de la deuxième entrée (6).

2. Dispositif de mesure (1) selon la revendication 1, **caractérisé en ce que** le module de sélection (13) est un module de sélection pneumatique.

3. Dispositif de mesure (1) selon la revendication 2, **caractérisé en ce que** le module de sélection pneumatique (13) comprend une électrovanne (14) comprenant deux entrées (15,16), respectivement reliées à la première (5) et à la deuxième (6) entrée du dispositif de mesure (1), et une sortie (17) en communication avec l'organe de mesure (3).

4. Dispositif de mesure (1) selon l'une quelconque des revendications 1 à 3, **caractérisé en ce qu'**il comprend un capteur de pression (10) agencé au niveau de la sonde au zirconium (4) et assujetti au microcontrôleur (9).

5. Module de séparation d'air (20) pour générateur de gaz d'inertage dans un système d'inertage d'au moins un réservoir de carburant d'un aéronef, le module présente intérieurement au moins une membrane perméable, et comprend une entrée (22) d'air comprimé destiné à traverser la membrane, une sortie (23) d'air enrichi en oxygène, et une sortie (21) d'air appauvri en oxygène dit gaz d'inertage, **caractérisé en ce qu'**il comprend un dispositif de mesure (1) conforme à l'une quelconque des revendications 1 à 4, dont la première entrée (5) est reliée à la sortie (21) d'air appauvri en oxygène du module de séparation d'air (20), et dont la deuxième entrée (6) est reliée ou destinée à être reliée à une source d'air ambiant (24).

## Patentansprüche

1. Vorrichtung (1) zur Messung der in einem zu analysierenden Gas vorhandenen Sauerstoffmenge, wobei die Vorrichtung (1) ein Gehäuse (2) umfasst, das mindestens ein Sauerstoffmengenmesselement (3) umfasst, das eine Zirkoniumsonde (4) umfasst, die geeignet ist, den in einem Gas vorhandenen Sauerstoffpartialdruck zu messen, wobei das Gehäuse (2) einen ersten Eingang (5), der dazu bestimmt ist, mit der Sonde (4) des Messelements (3) zur Versorgung mit dem zu analysierenden Gas in Verbindung zu stehen, einen Ausgang (7) für den Auslass des analysierten Gases, einen zweiten Eingang (6), der dazu bestimmt ist, mit der Sonde (4) des Messelements (3) in Verbindung zu stehen, wodurch es ermöglicht wird, die Vorrichtung (1) selektiv mit Umgebungsluft, die eine bekannte Sauerstoffmenge enthält, zu versorgen, so dass die Messung der in der Umgebungsluft vorhandenen Sauerstoffmenge es ermöglicht, eine potenzielle Abweichung der Messung des Messelements (3) im Verhältnis zur tatsächlichen bekannten Sauerstoffmenge in der Umgebungsluft zu bestimmen, das Messelement (3), **dadurch gekennzeichnet, dass** die Vorrichtung (1) einen Mikrocontroller (9) umfasst, der dem Messelement (3) unterliegt, um eine automatische Neukalibrierung des Messelements (3) in Abhängigkeit von der bestimmten Abweichung durchzuführen, wobei die Vorrichtung (1) einen Temperatursensor (18) umfasst, der an der Zirkoniumsonde (4) angeordnet ist und dem Mikrocontroller (9) unterliegt, so dass der Mikrocontroller (9) in der Lage ist, die Versorgungsspannung der Zirkoniumsonde (4) in Abhängigkeit von der gemessenen Temperatur zu verändern, wobei die Vorrichtung ferner ein Auswahlmodul (13) umfasst, das von dem Mikrocontroller (9) gesteuert wird und in der Lage ist, selektiv den ersten Eingang (5) oder den zweiten Eingang (6) mit dem Messelement (3) in Verbindung zu setzen, um entweder das von dem ersten Eingang (5) kommende Gas oder die von dem zweiten Eingang (6) kommende Umgebungsluft zu analysieren.

2. Messvorrichtung (1) nach Anspruch 1, **dadurch gekennzeichnet, dass** das Auswahlmodul (13) ein pneumatisches Auswahlmodul ist.

3. Messvorrichtung (1) nach Anspruch 2, **dadurch gekennzeichnet, dass** das pneumatische Auswahlmodul (13) ein Magnetventil (14) umfasst, das zwei Eingänge (15, 16), die jeweils mit dem ersten (5) und dem zweiten (6) Eingang der Messvorrichtung (1) verbunden sind, und einen Ausgang (17) umfasst, der mit dem Messelement (3) in Verbindung steht.

4. Messvorrichtung (1) nach einem der Ansprüche 1 bis 3, **dadurch gekennzeichnet, dass** sie einen Drucksensor (10) umfasst, der an der Zirkoniumsonde (4) angeordnet ist und dem Mikrocontroller (9) unterliegt.

5. Lufttrennmodul (20) für einen Inertgasgenerator in einem Inertisierungssystem mindestens eines Kraftstofftanks eines Flugzeugs, wobei das Modul innen mindestens eine durchlässige Membran aufweist und einen Eingang (22) für Druckluft, die dazu bestimmt ist, die Membran zu durchdringen, einen Ausgang (23) für sauerstoffangereicherte Luft und einen Ausgang (21) für sauerstoffabgereicherte Luft, das Inertgas, umfasst, **dadurch gekennzeichnet, dass** es eine Messvorrichtung (1) nach einem der Ansprüche 1 bis 4 umfasst, deren erster Eingang (5) mit dem Auslass (21) für sauerstoffabgereicherte Luft des Lufttrennmoduls (20) verbunden ist und dessen zweiter Eingang (6) mit einer Umgebungsluftquelle (24) verbunden ist oder dazu bestimmt ist, mit einer solchen verbunden zu werden.

## Claims

1. A device (1) for measuring the amount of oxygen present in a gas to be analysed, said device (1) comprising a casing (2) containing at least one oxygen amount measuring member (3) comprising a zirconium probe (4) able to measure the partial pressure of oxygen present in a gas, the casing (2) comprising a first inlet (5) for communicating with said probe (4) of the measuring member (3) for supplying the gas to be analysed, an outlet (7) for exhausting said gas analysed, a second inlet (6) for communicating with the probe (4) of the measuring member (3), for selectively supplying the device (1) with ambient air having a known amount of oxygen, such that the measurement of the amount of oxygen present in the ambient air enables the determination of any potential drift in the measurement from said measuring member (3) relative to the actual known amount of oxygen present in the ambient air, the measuring member (3), **characterised in that** the device (1) comprises a microcontroller (9) fastened to the measuring member (3) to perform automatic recalibration of the measuring member (3) based on the drift determined drift, the device (1) comprising a temperature sensor (18) arranged at the zirconium probe (4) and fastened to the microcontroller (9), so that the microcontroller (9) is able to vary the supply voltage to the zirconium probe (4) as a function of the temperature measured, the device further comprises a selection module (13) controlled by the microcontroller (9) and able to communicate either the first inlet (5) or the second inlet (6) with the measuring member (3) to analyse either the gas derived from the first inlet (5) or the ambient air derived from the second inlet (6).

2. The measuring device (1) according to claim 1, **characterised in that** the selection module (13) is a pneumatic selection module.

3. The measuring device (1) according to claim 2, **characterised in that** the pneumatic selection module (13) comprises a solenoid valve (14) comprising two inlets (15, 16), respectively connected to the first (5) and second (6) inlet of the measuring device (1), and an outlet (17) in communication with the measuring member (3).

4. The measuring device (1) according to any one of claims 1 to 3, **characterised in that** it comprises a pressure sensor (10) arranged at the zirconium probe (4) and fastened to the microcontroller (9).

5. An air separation module (20) for an inerting gas generator in an inerting system of at least one fuel tank of an aircraft, the module has internally at least one permeable membrane, and comprises an inlet (22) for compressed air intended to pass through the membrane, an outlet (23) of oxygen-enriched air, and an outlet (21) of oxygen-depleted air called inerting gas, **characterised in that** it comprises a measuring device (1) in accordance with any one of claims 1 to 4, the first inlet (5) of which is connected to the oxygen-depleted air outlet (21) of the air separation module (20), and the second inlet (6) of which is connected to or intended to be connected to an ambient air source (24).
